# EUROPÄISCHE PATENTANMELDUNG

(11) **EP 0 841 404 A1**
(43) Veröffentlichungstag der Anmeldung: **13.05.1998**
(21) Anmeldenummer: 97118365.2
(22) Anmeldetag: 22.10.1997
(51) Int. Cl.: C12Q 1/58

(54) **Testeinrichtung zum Harnstoffnachweis in Milch**

(30) Priorität: 08.11.1996 AT 1957/96
(71) Anmelder: Buchrucker, Karlheinz, Mag., 4100 Ottensheim (Oberösterreich) (AT)
(72) Erfinder: Buchrucker, Karlheinz, Mag., 4100 Ottensheim (Oberösterreich) (AT)
(74) Vertreter: VOSSIUS & PARTNER

(57) **Zusammenfassung**

Eine Testeinrichtung zum Harnstoffnachweis in Milch besteht aus einer Handhabe, die eine Testsubstanz mit Urease als Reagens und einem Indikatorfarbstoff zur Anzeige der reaktionsbedingten pH-Wert-Verschiebung aufnimmt.

Um einen einfachen, stalltauglichen und ausreichend genauen Harnstofftest durchführen zu können, ist eine pulverförmige Mischung aus einem natürlichen organischen Urease-Trägerstoff, einem inerten Antibackmittel und einem Indikatorfarbstoff als Testsubstanz in einen die Handhabe bildenden Probenbehälter eingebracht, welcher durchsichtige Probenbehälter einen öffenbaren Verschluß zum Einfüllen einer Milchprobe aufweist.

## Beschreibung

Die Erfindung bezieht sich auf eine Testeinrichtung zum Harnstoffnachweis in Milch, bestehend aus einer Handhabe, die eine Testsubstanz mit Urease als Reagens und einem Indikatorfarbstoff zur Anzeige der reaktionsbedingten pH-Wert-Verschiebung aufnimmt.

Harnstoff ist das mengenmäßig wichtigste Abbauprodukt des Eiweiß-Stoffwechsels und die Bestimmung des Harnstoffgehalts der Milch laktierender Kühe bietet eine praxisgerechte Orientierung zur Abstimmung der Energie- bzw. Einweißversorgung der Tiere, so daß sich Fütterungsfehler, die bei längerer Dauer vor allem die Gesundheit und Fruchtbarkeit der Kuh beeinträchtigen, erkannt und korrigiert werden können.

Verfahren zum Nachweis von Harnstoff in Körperflüssigkeiten sind im klinischen Bereich gut bekannt, wobei eine dieser Methoden auf der chemischen Hydrolyse beruht, eine andere auf der direkten colormetrischen Reaktion von Harnstoff im proteinfreien Filtrat mit einem organischen Reagens, wie Diazetyl Monoxime, und eine weitere Methode auf dem enzymatischen Abbau von Harnstoff durch Urease zu einem Ammoniumsalz und dessen Bestimmung mittels Titration, wobei alle diese Methoden entsprechende Fachkenntnisse erfordern, einen beträchtlichen apparativen Aufwand mit sich bringen und für Routineuntersuchungen vor allem im Stall kaum verfügbar sind.

Gemäß der GB-PS 922 665 ist auch schon ein Schnelltest zur spezifischen enzymatischen Bestimmung des Harnstoffgehaltes im Blut bekannt, wobei eine Testeinrichtung aus einem Teststreifen Verwendung findet, dessen Reaktionszone mit Urease als Reagens sowie Bromthymolblau als Indikator imprägniert und mit einer semipermeablen Membrane überzogen ist. Nach dem Benetzen mit Blut diffundiert der im Blut enthaltene Harnstoff durch die Membrane und wird auf Grund des Enzyms Urease in Ammoniak und Kohlendioxyd gespalten. Ammoniak bildet in wässriger Lösung Ammoniumhydroxyd, das den pH-Wert erhöht, und diese pH-Wert-Verschiebung läßt sich durch eine Farbänderung des Indikators Bromthymol-blau von grün-gelb in verschiedenen Abstufungen bis grün-blau anzeigen. Der für Blut entwickelte Harnstoffnachweis wurde auch schon für die Abwendung bei Milch adaptiert, da wissenschaftliche Untersuchungen die enge Beziehung zwischen Blut- und Milchharnstoffgehalten nachgewiesen haben. Allerdings erfordern diese bekannten Testeinrichtungen eine Einwirkzeit von mindestens 4 Minuten und dann eine sofortige Ablesung und Auswertung der Farbreaktion, da sich die Farben in der Reaktionszone rasch verändern. Dazu kommen noch die recht begrenzte Haltbarkeit, die kleine Reaktionsfläche der Reaktionszonen, die vielstückigen Abpackungen, der verhältnismäßig hohe Preis und nicht zuletzt die beschränkte Verfügbarkeit über Terärzte und Apotheken, so daß die milchgeeigneten Harnstofftests bisher nicht befriedigen.

Der Erfindung liegt daher die Aufgabe zugrunde, diese Mängel zu beseitigen und eine Testeinrichtung der eingangs geschilderten Art zu schaffen, die einen stalltauglichen, leicht durchzuführenden, empfindlichen, schnellen, aussagekräftigen, lagerfähigen und preisgünstigen Harnstoffnachweis als Suchtest für Fütterungsfehler laktierender Kühe erlaubt.

Die Erfindung löst diese Aufgabe dadurch, daß eine pulverförmige Mischung aus einem natürlichen organischen Urease-Trägerstoff, vorzugsweise Schwertbohnenmehl, einem inerten Antibackmittel, vorzugsweise Aerosil oder Polyurethanschaumstoffmehl, und einem Indikatorfarbstoff, vorzugsweise Phenolrot oder Bromthymolblau, als Testsubstanz in einen die Handhabe bildenden Probenbehälter eingebracht ist, welcher durchsichtige Probenbehälter, vorzugsweise Probenröhrchen, einen öffenbaren Verschluß zum Einfüllen einer Milchprobe aufweist. Zur Überprüfung des Harnstoffgehaltes in der Milch braucht lediglich eine Milchprobe in den Probenbehälter eingefüllt und mit der sich darin befindenden Testsubstanz durch Schütteln vermischt zu werden, wobei das Antibackmittel ein Zusammenbacken der pulverförmigen Substanzen bei der Milchzugabe verhindert, und auf Grund des ureasebedingten Harnstoffabbaues kommt es nach ca. 3 Minuten zu einer entsprechenden Farbreaktion des Indikatorfarbstoffes und damit zu einer Einfärbung der Milchprobe selbst, so daß sich eine gute Vergleichsmöglichkeit der Milchprobenfärbung mit einer geeigneten Auswert-Farbskala ergibt. Da jeder Probenbehälter eine geschlossene Einheit darstellt und die Entnahme eines Behälters nicht die Lagerfähigkeit der restlichen Behälter beeinträchtigt, werden lange Lagerbeständigkeiten erreicht. Außerdem ist die Beständigkeit der Urease im natürlichen Trägerstoff wesentlich größer als die isolierte Reinsubstanz und die Farbreaktion bleibt über einen längeren Zeitraum erhalten.

Übliche Probenbehälter weisen ein Aufnahmevolumen von 10 ml auf und enthalten ca. 450 mg pulverförmige Mischung der Testsubstanz, die sich im Mengenverhältnis 2 : 1 aus Urease-Trägerstoff und Antibackmittel und einem 0,2 Gew.%igen Anteil eines den Reaktionsbereich erfassenden Indikatorfarbstoffes zusammensetzt. Die erfindungsgemäße Testeinrichtung erlaubt einen für jeden auch ohne Fachkenntnis einwandfrei durchführbaren Harnstoffnachweis mit einer für das Aufdecken von Fütterungsfehlern ausreichenden semiquantitativen Feststellung des Harnstoffgehaltes der Milch.

Um das Vermischen der Testsubstanz mit der eingefüllten Milchprobe zu verbessern, nimmt der Probenbehälter wenigstens eine Mischkugel auf und bildet der Behälterverschluß einen zum Behälterinneren hin offenen Hohlraum. Durch den Hohlraum entsteht auch bei einer über die Füllmarkierung hinausgehenden Befüllung des Probenbehälters eine Luftblase innerhalb des verschlossenen Behälters, die das Schütteln erleichtert und zusammen mit der Rührwirkung der Mischkugel zu einer schnellen und homogenen Verteilung der Testsubstanz innerhalb der Milch führt.

Gibt es eine Verpackungseinheit aus einer Schachtel mit einigen, vorzugsweise vier. Probenbehältern, wobei an der Innenseite des Schachteldeckels ein Farbschema zur Auswertung eines durchgeführten Tests vorgesehen ist, wird auch den Bedürfnissen kleiner Betriebe Rechnung getragen, da diese vergleichsweise geringe Zahl der Probenbehälter und die entsprechend lange Lagerfähigkeit eine gefahrlose Nutzung der vorrätigen Probenbehälter erlauben, wobei die Schachtel nicht nur zur Aufbewahrung der Probenbehälter, sondern auch gleich als Träger für das Farbschema zur Probenauswertung dient.

## Patentansprüche

1. Testeinrichtung zum Harnstoffnachweis in Milch, bestehend aus einer Handhabe, die eine Testsubstanz mit Urease als Reagens und einem Indikatorfarbstoff zur Anzeige der reaktionsbedingten pH-Wert-Verschiebung aufnimmt, dadurch gekennzeichnet, daß eine pulverförmige Mischung aus einem natürlichen organischen Urease-Trägerstoff, vorzugsweise Schwertbohnenmehl, einem inerten Antibackmittel, vorzugsweise Aerosil oder Polyurethanschaumstoffmehl, und einem Indikatorfarbstoff, vorzugsweise Phenolrot oder Bromthymolblau, als Testsubstanz in einen die Handhabe bildenden Probenbehälter eingebracht ist, welcher durchsichtige Probenbehälter, vorzugsweise Probenröhrchen, einen öffenbaren Verschluß zum Einfüllen einer Milchprobe aufweist.

2. Testeinrichtung nach Anspruch 1, dadurch gekennzeichnet, daß der Probenbehälter wenigstens eine Mischkugel aufnimmt und der Behälterverschluß einen zum Behälterinneren hin offenen Hohlraum bildet.

3. Testeinrichtung nach Anspruch 1 oder 2, gekennzeichnet durch eine Verpackungseinheit aus einer Schachtel mit einigen, vorzugsweise vier, Probenbehältern, wobei an der Innenseite des Schachteldeckels ein Farbschema zur Auswertung eines durchgeführten Tests vorgesehen ist.
